# EUROPEAN PATENT SPECIFICATION

(11) **EP 1 698 676 B1**
(45) Date of publication and mention of the grant of the patent: **05.01.2011**
(21) Application number: 06250605.0
(22) Date of filing: 03.02.2006
(51) Int. Cl.: C09J 183/04, A61K 8/89

(54) **Pressure-sensitive adhesive composition applicable to nail and adhesive agent for nail**
Druckempfindliche Klebstoffzusammensetzung für Nägel
Composition adhésive sensible à la pression pour les ongles

(30) Priority: 03.02.2005 JP 2005028283
(43) Date of publication of application: 06.09.2006
(73) Proprietor: NITTO DENKO CORPORATION, Ibaraki-shi, Osaka 567-8680 (JP)
(72) Inventor: Hanatani, Akinori, c/o Nitto Denko Corporation, Ibaraki-shi, Osaka 567-8680 (JP); Akemi, Hitoshi, c/o Nitto Denko Corporation, Ibaraki-shi, Osaka 567-8680 (JP)
(74) Representative: Benson, John Everett

(56) References cited:
- EP-A- 1 329 225
- WO-A-00/74661
- WO-A-01/52823
- WO-A-03/020248
- WO-A-2004/050375
- US-A- 4 655 767

## Description

### FIELD OF THE INVENTION

The present invention relates to a pressure-sensitive adhesive composition to be used on adhesion to a nail plate and an adhesive agent for nail comprising the composition.

### BACKGROUND ART

Nail adhesive (JP-B-2648735), lacquer (JP-B-2635104) and the like have been proposed for use for fixing artificial nail, nail plate protector, ingrown nail corrector etc., therapeutic agents for onychomycosis, hapalonychia, onycholysis, onychophagia and the like, and nailcare products and the like for cosmetic purposes. However, volatile organic solvents contained therein and chemical agents contained in polish remover etc. used for removing them from the nail may damage the nail and the surrounding skin, and inhalation of vapor thereof may cause poisoning symptoms.

To deal with this, a pressure-sensitive adhesive composition applicable for nail, which contains an adhesive, and an adhesive agent for nail comprising the composition have been proposed (JP-A-10-330247). They are advantageous in that they are free of solvents, and do not require use of chemical agents for removal from the nail.

### SUMMARY OF THE INVENTION

The above-mentioned pressure-sensitive adhesive composition applicable for nail is required to have a superior adhesive force and cohesion. This is because nails should often bear load during walking, grabbing objects and the like and the composition should not fall off easily. It is needless to say that the absence of plaster remaining on the nail plate (i.e., glue remainder) upon removal of the pressure-sensitive adhesive composition is preferable.

However, the pressure-sensitive adhesive compositions applicable for nail, which have heretofore been reported, fail to ensure sufficient adhesive performance.

Moreover, the treatment of onychomycosis requires supply of an antifungal agent in an amount sufficient to achieve a bacteriostatic effect on a nail plate for a long time. This in turn necessitates not only adhesive performance permitting adhesion for a long time but also high drug releaseability and drug stability.

It is therefore an object of the present invention to provide a pressure-sensitive adhesive composition applicable for nail, which has a superior adhesive force and cohesion, and further, superior drug releasability and drug stability, and an adhesive agent for nail comprising the composition.

The present inventors have conducted intensive studies in an attempt to achieve the above-mentioned objects and found that use of a pressure-sensitive adhesive composition containing a specific condensation polymer can solve the above-mentioned problems, which resulted in the completion of the present invention. Accordingly, the present invention provides the following.
[1] A pressure-sensitive adhesive composition applicable for nail, which comprises a condensation polymer of a silicone resin and a silicone rubber.
[2] The composition of [1], which comprises not less than two kinds of condensation polymers having different silicone resin/silicone rubber ratios.
[3] The composition of [1] or [2], wherein the condensation polymer has a silicone resin/silicone rubber ratio of 35/65 - 70/30 (w/w).
[4] The composition of [3], wherein the condensation polymer and an acrylic adhesive are mixed and crosslinked.
[5] The composition of any of [1]-[5], further comprising at least one kind of additive selected from silicone oil, fatty acid ester of monohydric alcohol, fatty acid ester of polyol and higher alcohol.
[6] The composition of any of [1]-[5], further comprising a drug.
[7] The composition of [6], wherein the condensation polymer has a residual silanol concentration of less than 800 ppm.
[8] The composition of [7], comprising not less than two kinds of condensation polymers having different residual silanol concentrations.
[9] The composition of any of [6]-[8], further comprising an organic acid.
[10] The composition of [9], wherein the organic acid is tartaric acid or salicylic acid.
[11] An adhesive agent for nail, which comprises a support and an adhesive layer comprising a composition of any of [1]-[10], which is formed on one surface of the support.

The pressure-sensitive adhesive composition applicable for nail of the present invention has a superior adhesive force and cohesion. When an adhesive agent comprising this composition is adhered to a nail plate, it does not fall off easily, and when it is peeled off, a glue remainder does not occur easily.

Furthermore, the pressure-sensitive adhesive composition applicable for nail of the present invention has superior drug releasability and drug stability. Therefore, it can be preferably used as an adhesive agent aiming at the treatment of onychomycosis, hapalonychia, onycholysis, onychophagia and the like.

### DETAILED DESCRIPTION OF THE INVENTION

In the following, the pressure-sensitive adhesive composition applicable for nail of the present invention is also referred to as the composition of the present invention. The composition of the present invention comprises a condensation polymer of a silicone resin and a silicone rubber. In the following, unless otherwise specified, a "condensation polymer" means a condensation polymer of a silicone resin and a silicone rubber.

The composition of the present invention may contain a condensation polymer alone, or may further contain other adhesives, the below-mentioned drugs and various additives depending on the object. The content of the total adhesives in the composition of the present invention is preferably not less than 50 wt%, more preferably not less than 70 wt%, to exert the adhesive performance and the like to the necessary and sufficient extent. As used herein, the content of the total adhesives refers to the total amount of a condensation polymer, and, if present, other adhesives such as the below-mentioned acrylic adhesive and the like.

The condensation polymer to be used in the present invention is obtained by dehydrative condensation and the like of a mixture of a silicone resin and a silicone rubber in the presence of, for example, an alkaline catalyst etc., and the condensation polymer is superior in the adhesive performance, cohesion and stability during production.

The constitution ratio of a silicone resin and a silicone rubber to give a condensation polymer is not particularly limited. To achieve balanced adhesive performance as a pressure-sensitive adhesive composition applicable for nail, the weight ratio of silicone resin/silicone rubber is preferably 35/65 - 70/30 (w/w), more preferably 40/60 - 65/35 (w/w). When the above-mentioned weight ratio is high, cohesion becomes strong but tackiness tends to become weak. As a result, the adhesive force becomes strong but the adhesiveness may be lost. When the above-mentioned weight ratio is small, cohesion and adhesive force tend to be weak, which may disqualify the pressure-sensitive adhesive composition applicable for nail.

Here, the silicone rubber is a long chain polymer having OH group on both terminals of polyorganosiloxane and, for example, represented by the formula: (OH)R₂Si(R₂SiO_{1/2})ₙOSiR₂OH, wherein R is hydroxyl group, monovalent hydrocarbon group (e.g., methyl, phenyl and the like) or -Si(R¹)₃ (R¹ is monovalent hydrocarbon group such as methyl, phenyl and the like), and n is any positive integer.

As the organosiloxane unit of silicone rubber, dimethylsiloxane, diphenylsiloxane and the like can be mentioned. Not less than two kinds of organosiloxane units may be used in combination. In consideration of the adhesive properties such as adhesive force, tackiness and the like, a silicone rubber comprising dimethylsiloxane as a main component is preferable.

Here, as a silicone resin, conventionally known ones can be used without any particular limitation. As the silicone resin, for example, MQ resin which is a polymer having a three-dimensional structure consisting of M unit represented by R₃SiO_{1/2} (R is as defined above) and Q unit represented by SiO_{4/2}; DT resin consisting of D unit represented by R₂SiO_{2/2} (R is as defined above) and T unit represented by RSiO_{3/2} (R is as defined above); DTQ resin consisting of D unit, T unit and Q unit and the like can be mentioned. In consideration of the adhesive properties such as adhesive force, tackiness and the like, MQ resin is preferable.

The constitution ratio of M unit and Q unit in the MQ resin is not particularly limited. To provide balanced adhesive performance of a pressure-sensitive adhesive composition applicable for nail, the amount of M unit (molar ratio) relative to Q unit is preferably 5/10 - 12/10, more preferably 6/10 - 9/10. When the above-mentioned molar ratio is high, cohesion and adhesive force tend to become weak. When the above-mentioned molar ratio is small, the tackiness tends to become weak, and a superior pressure-sensitive adhesive composition applicable for nail may not be obtained.

A silicone resin and a silicone rubber can be condensed according to a method known per se, such as a method described in JP-B-3538328, using an alkali catalyst and the like.

The condensation polymer obtained as mentioned above contains residual silanol group not involved in polymerization or condensation reaction.

When the composition of the present invention further contains a drug such as the below-mentioned antifungal agent etc., the interaction between the residual silanol and the drug has been found to possibly degrade drug releasability strikingly. Therefore, it is preferable to blbck the residual silanol group with trimethylsilyl group etc. to suppress the residual silanol concentration of the condensation polymer. The present inventors have found that a pressure-sensitive adhesive composition superior in drug releasability can be obtained by the use of such a condensation polymer.

The residual silanol group can be blocked by a method known per se, for example, a method described in JP-A-2000-229885, trimethylsilylation and the like, without any particular limitation.

The upper limit of the residual silanol concentration of the condensation polymer to be used in the present invention is preferably less than 800 ppm, more preferably less than 200 ppm.

When the residual silanol concentration is not less than 800 ppm, releasability may be degraded strikingly depending on the drug, as mentioned above.

The lower limit of the residual silanol concentration is not particularly limited. When the residual silanol concentration is suppressed too much, drug stability may be degraded. It is preferably not less than 10 ppm, more preferably 20 ppm.

Here, the residual silanol concentration (ppm) is a numerical value defined by Kellum et al., ANAL. CHEM., 39, 1623 (1967) and JORDAN, ANAL. CHEM., 30, 297 (1964), and can be determined by titration using lithium aluminum hydride di-N-butylamide.

The above-mentioned preferable condensation polymer is commercially available in the form of a solution, and such commercially available product can be selected and used as appropriate. As the commercially available product, for example, BIO-PSA 7-4502 [resin/rubber ratio: 60/40 (w/w), residual silanol concentration: about 600 ppm], BIO-PSA7-4602 [resin/rubber ratio: 55/45 (w/w), residual silanol concentration: about 600 ppm], BIO-PSA 7-4402 [resin/rubber ratio: 65/35 (w/w), residual silanol concentration: about 600 ppm], BIO-PSA 7-4202 [resin/rubber ratio: 60/40 (w/w), residual silanol concentration: about 125 ppm], BIO-PSA 7-4102 [resin/rubber ratio: 65/35 (w/w), residual silanol concentration: about 125 ppm], BIO-PSA 7-4302 [resin/rubber ratio: 55/45 (w/w), residual silanol concentration: about 125 ppm] manufactured by Dow Corning Corporation, and the like can be mentioned.

Here, the "resin/rubber ratio" is a weight ratio of a silicone resin and a silicone rubber to give a condensation polymer.

To achieve optimally balanced adhesive performance as the composition of the present invention, the adhesive performance can be fine-tuned by appropriately mixing two or more kinds of condensation polymers having different resin/rubber ratios. Similarly, two or more kinds of MQ resins having different constitution ratios of M unit and Q unit may be appropriately mixed.

Furthermore, to afford the optimal balance between drug releasability and drug stability, it is more preferable to appropriately mix two or more kinds of condensation polymers having different residual silanol concentrations to fine-tune the residual silanol concentration.

To increase cohesion of the composition of the present invention, the condensation polymer may form a crosslinking structure. The crosslinking method is not particularly limited, and a method comprising dehydrative condensation of residual silanol, a method comprising radical condensation using organic peroxide as a crosslinking agent, a method comprising addition reaction in the presence of a platinum catalyst etc., using modified silicone having vinyl group, alkoxy group and the like as a condensation polymer and adding a crosslinking agent having hydrosilyl group and the like can be mentioned.

In view of the fact that, when a drug is added as mentioned below, the drug may become a catalyst poison of platinum catalyst etc. and the like, a method using organic peroxide is preferable.

As the organic peroxide to be used as a crosslinking agent, a conventionally known one can be used without any particular limitation.

For example, benzoyl peroxide (BPO), t-butyl peroxybenzoate, dicumyl peroxide, t-butylcumyl peroxide, t-butyloxide, 2,5-dimethyl-2,5-di-t-butylperoxyhexane, 2,4-dichloro-benzoyl peroxide, di-t-butylperoxy-diisopropylbenzene, 1,1-bis(t-butylperoxy)-3,3,5-trimethylcyclohexane, 2,5-dimethyl-2,5-di-t-butylperoxyhexyne-3 and the like can be mentioned.

These organic peroxides may be used in combination of two or more kinds thereof. The amount of organic peroxide to be used is preferably about 0.2 - 5 parts by weight per 100 parts by weight of the condensation polymer.

As the crosslinking agent having hydrosilyl group, a conventionally known one can be used without any particular limitation. For example, polymethyl hydrogen siloxane and the like can be mentioned.

While the amount of crosslinking agent having hydrosilyl group to be used varies depending on the content ratio of vinyl group etc. in the condensation polymer, it is generally preferably about 5 - 50 parts by weight per 100 parts by weight of condensation polymer.

As the metal crosslinking agent to be used together with the catalyst having hydrosilyl group, a transition metal catalyst is preferable, and a platinum catalyst such as platinum, platinate chloride etc. is particularly preferably used. The amount of the metal catalyst to be used is preferably about 0.001 - 1.5 parts by weight per 100 parts by weight of condensation polymer.

The composition of the present invention can improve cohesion of the composition as a whole by forming a crosslinking structure by further adding an acrylic adhesive to the condensation polymer.

In the present invention, "condensation polymer and an acrylic adhesive are mixed and crosslinked" includes, for example, all of the embodiments wherein 1) the condensation polymer is crosslinked and the acrylic adhesive is not crosslinked; 2) the condensation polymer is not crosslinked and the acrylic adhesive is crosslinked; 3) the condensation polymer and the acrylic adhesive are crosslinked, and the like. To achieve the desired cohesion, the embodiment of 2) or 3) is preferable.

Each of these embodiments can be achieved by mixing a condensation polymer, an acrylic adhesive and the desired crosslinking agent, and heating etc. to allow crosslinking of the composition as a whole.

As the acrylic adhesive, one having a functional group such as carboxyl group etc. is preferable. Specifically, a copolymer of alkyl (meth)acrylate and a comonomer containing a functional group, which is copolymerizable therewith, is preferable.

Alkyl (meth)acrylate containing alkyl group having 4 to 18 carbon atoms is preferable. Particularly, 2-ethylhexyl (meth)acrylate, butyl (meth)acrylate, isobutyl (meth)acrylate, hexyl (meth)acrylate, octyl (meth)acrylate, isooctyl (meth)acrylate, decyl (meth)acrylate, isodecyl (meth)acrylate, lauryl (meth)acrylate, stearyl (meth)acrylate and the like can be preferably used. These compounds may be used alone or two or more kinds thereof may be used in combination.

As the functional group-containing comonomer, a monomer having hydroxyl group, a monomer having carboxyl group, a monomer having amide group, a monomer having amino group and the like can be mentioned. As the monomer having hydroxyl group, hydroxyalkyl (meth)acrylate such as 2-hydroxyethyl (meth)acrylate, 2-hydroxypropyl (meth)acrylate and the like, and the like are preferable. As the monomer having carboxyl group, monoalkyl maleate ester such as butyl maleate etc., maleic acid, maleic anhydride, fumaric acid, crotonic acid and the like are preferable. As the monomer having amide group, alkyl(meth)acrylamide such as dimethylacrylamide, diethylacrylamide etc.; alkylethermethylol(meth)acrylamide such as butoxymethylacrylamide, ethoxymethylacrylamide etc.; diacetoneacrylamide, N-vinyl-2-pyrrolidone and the like are preferable, and as the monomer having amino group, dimethylaminoethylacrylate is preferable.

The amount of copolymerization of the functional group-containing comonomer in the copolymer is preferably not more than 50 wt% of the whole acrylic copolymer.

The polymerization method is not particularly limited and, for example, solution polymerization, emulsion polymerization, mass polymerization, suspension polymerization and the like can be mentioned.

The weight average molecular weight of the acrylic adhesive is not particularly limited, but in consideration of adhesive properties such as adhesive force, tackiness and the like, it is preferably 100,000 - 5,000,000, more preferably 200,000 - 3,000,000.

An acrylic adhesive satisfying such conditions is commercially available in the form of a solution, and can be selected from commercially available products without any limitation. As the commercially available acrylic adhesive, for example, DURO-TAK 87-2196, DURO-TAK387-2516 and the like manufactured by NATIONAL STARCH & CHEMICAL can be mentioned, and two or more kinds thereof may be used in combination.

The ratio of a condensation polymer and an acrylic adhesive in the composition is not particularly limited, but in consideration of the adhesive properties such as adhesive force, tackiness and the like, condensation polymer/acrylic adhesive (weight ratio) is preferably 10/90 - 80/20 (w/w), more preferably 20/80 - 70/30 (w/w). When the aforementioned ratio is small, the composition tends to show insufficient cohesion, and when the aforementioned ratio is high, the composition may not exhibit superior adhesive performance relative to a nail plate.

As the crosslinking agent for acrylic adhesive, polyfunctional isocyanate, metal chelate compound and the like can be mentioned. As the crosslinking agent for condensation polymer, the aforementioned organic peroxide, crosslinking agent having hydrosilyl group and the like can be mentioned. Two or more kinds thereof may be used in combination.

Specific examples of the above-mentioned polyfunctional isocyanate include tolylene diisocyanate, 4,4'-diphenylmethanediisocyanate, hexamethylene diisocyanate, m-xylene diisocyanate, p-xylene diisocyanate, 1,5-naphthalene diisocyanate, isophorone diisocyanate, lysin diisocyanate, hydrogenated 4,4'-diphenylmethane diisocyanate, hydrogenated tolylene diisocyanate and the like, and as the metal chelate compound, aluminum triacetylacetonate, titanium triacetylacetonate and the like can be mentioned.

The polyfunctional isocyanate is added in an amount of preferably 0.1 - 2 parts by weight per 100 parts by weight of the total of the condensation polymer and the acrylic adhesive.

In consideration of adjustment of adhesive force and cohesion, and improvement of drug permeability, the composition of the present invention preferably further comprises at least one kind of additive selected from silicone oil, fatty acid ester of monohydric alcohol, fatty acid ester of polyol and higher alcohol, where silicone oil and fatty acid ester of polyol are more preferable.

Two or more kinds of these additives may be used in combination. The additive is contained in the composition in a proportion of preferably not more than 40 parts by weight, more preferably 2 - 30 parts by weight, per 100 parts by weight of the total of the condensation polymer and an acrylic adhesive (when present). When the amount of the additive is large, the cohesion tends to decrease, or cohesive failure or glue remainder may occur, and furthermore, plaster may flow out easily during preservation (i.e., cold flow). Conversely, when the amount of the additive is small, the desired effect may not be achieved.

As the fatty acid ester of monohydric alcohol, an ester of straight chain or branched chain fatty acid having 6 to 20, preferably 6 to 16, carbon atoms, and straight chain or branched chain monohydric alcohol having 1 to 5, preferably 1 to 4, carbon atoms is preferable and, for example, isopropyl myristate, diethyl sebacate, isopropyl palmitate, diisopropyl adipate and the like can be mentioned, with preference given to isopropyl myristate.

As the fatty acid ester of polyhydric alcohol, an ester of straight chain or branched chain fatty acid having 6 to 16, preferably 8 to 10, carbon atoms and straight chain or branched chain polyol having 2 to 10, preferably 2 to 6, carbon atoms is preferable and, for example, propyleneglycol dicaprylate, propyleneglycol monocaprylate, propyleneglycol dicaprate, ethyleneglycol monocaprylate, ethyleneglycol diisooctanoate, glycerol monocaprylate, sorbitan monocaprylate and the like can be mentioned, with preference given to propyleneglycol dicaprylate.

As the higher alcohol, straight chain or branched chain alcohol having 8 to 24, preferably 10 to 22, carbon atoms is preferable and, for example, 2-octyldodecanol, decanol, lauryl alcohol, myristyl alcohol, cetyl alcohol, stearyl alcohol and the like can be mentioned, with preference given to 2-octyldodecanol.

The composition of the present invention can contain various drugs according to the object of treatment.

As the drug, any compound showing activity as a pharmaceutical agent by penetration through the nail plate, or absorption into the body through the nail plate can be used without any particular limitation. For example, the compounds of the following A) - R) and pharmaceutically acceptable salts thereof can be mentioned, which can be used in combination of two or more kinds thereof as necessary.
A) Antifungal agents; for example neticonazole, bifonazole, clotrimazole, miconazole, econazole, oxiconazole, itraconazole, fluconazole, thioconazole, isoconazole, sulconazole, croconazole, ketoconazole, lanoconazole, omoconazole, terconazole, sertaconazole, tolnaftate, terbinafine, butenafine, amorolfine, griseofulvin, ciclopirox, pentamycin, amphotericin B, pyrrolenitrin, clotrimazole and the like
B) Corticosteroids; for example, hydrocortisone, prednisolone, beclometasone propionate, flumethasone, triamcinolone, triamcinoloneacetonide, fluocinolone, fluocinoloneacetonide, fluocinoloneacetonide acetate, clobetasol propionate and the like
C) Analgesics, antiphlogistics; for example, acetaminophen, mefenamic acid, flufenamic acid, indomethacin, diclofenac, diclofenac sodium, alclofenac, oxyphenbutazone, phenylbutazone, ibuprofen, flurbiprofen, salicylic acid, methyl salicylate, 1-menthol, camphor, sulindac, tolmetin sodium, naproxen, fenbufen and the like
D) Hypnotic analgesics; for example, phenobalbital, amobarbital, cyclobalbital, triazolam, nitrazepam, lorazepam, haloperidol and the like
E) Neuroleptics; for example, fluphenazine, theolitazine, diazepam, fludiazepam, flunitrazepam, chlorpromazine and the like
F) Antihypertensive agents; for example, clonidine, pindolol, propranolol, bufuranolol, indenolol, nivadipine, lofedixine, nipradilol, bucumolol, nifedipine and the like
G) Hypotensive diuretics; for example, hydrothiazide, pendflunathiazide, cyclobenthiazide and the like
H) Antibiotics; for example, penicillin, tetracycline, oxytetracycline, fradiomycin, erythromycin, chloramphenicol and the like
I) Anesthetics; for example, lidocain, benzocain, ethyl aminobenzoate and the like
J) Antibacterial substances; for example, benzalkonium, nitrofurazone, nystatin, acetosunlfamine, clotrimazole and the like
K) Vitamins; for example, vitamin A, ergocalciferol, cholecalciferol, octotiamine, riboflavine tetrabutyrate and the like
L) Anticonvulsants; for example, nitrazepam, meprobamate, clonazepam and the like
M) Coronary vasodilators; for example, nitroglycol, isosorbide dinitrate, erythritol tetranitrate, pentaerythritol tetranitrate, propatyl nitrate and the like
N) Antihistamines; for example, diphenhydramine, chlorpheniramine, diphenylimidazole and the like
O) Antitussives; for example, dextromethorphan, terbutaline, ephedrine, salbutamol and the like
P) Sex hormones; for example, progesterone, estradiol and the like
Q) Antideprssants; for example, doxepin and the like,
R) Others; for example, 5-fluorouracil, dihydroergotamine, fentanyl, desmopressin, digoxin, metoclopramide, domperidone, Scopolamin, prostaglandin and the like.

The amount of the drug in the composition of the present invention can be appropriately determined according to the drug to be used and object of the treatment. It is preferably about 2 - 30 wt% of the total amount of the composition. When the amount of the drug is too high, the adhesive performance may be impaired.

When the composition of the present invention contains a drug, it is preferable to further contain an organic acid to improve drug stability of the composition.

The organic acid is not particularly limited and, for example, tartaric acid (L-(+)-tartaric acid, D-(+)-tartaric acid, racemic tartaric acid etc.), salicylic acid, citric acid, succinic acid, lactic acid, fumaric acid, benzoic acid, capric acid, caprylic acid, lauric acid, myristic acid, palmitic acid, stearic acid, isostearic acid, maleic acid, malonic acid, malic acid, adipic acid, sorbic acid and the like can be mentioned, with preference given to tartaric acid and salicylic acid.

The organic acid is contained in the composition in a proportion of preferably about 0.05 - 5 wt%, preferably 0.1 - 3 wt%.

The composition of the present invention may further contain various additives generally used in the field of pharmaceutical preparation, such as tackifier, pigment, solubilizer, drug absorption promoter, stabilizer, binder, filler, nail softener, moisturizer, cold flow preventer (e.g., metal salt of fatty acid such as calcium stearate, polysaccharide etc.) and the like, within the range that does not impair the effect of the present invention.

These additives can be used in combination of two or more kinds thereof. While the amount of addition is not particularly limited, addition of a large amount thereof causes insufficient cohesion, easily resulting in cohesive failure and glue remainder, and furthermore, a plaster layer may flow out easily during preservation (i.e., cold flow). Therefore, the total amount of additives is preferably about 1 - 20 wt% of the total amount of the composition.

An adhesive agent for nail can be obtained by forming the aforementioned composition of the present invention on one surface of the support as an adhesive layer.

In the following, the adhesive agent for nail of the present invention is also simply referred to as the adhesive agent of the present invention.

The production method of the adhesive agent of the present invention is not particularly limited and, for example, a method comprising dissolving or dispersing the composition of the present invention in a solvent such as toluene and the like, applying the obtained solution or dispersion on one surface of the support, and drying where necessary by heating to progress crosslinking reaction, thereby forming an adhesive layer on the surface of the support and the like can be mentioned. Alternatively, the adhesive agent can also be produced by applying the above-mentioned solution or dispersion on a release liner, drying where necessary by heating to progress crosslinking reaction, thereby forming an adhesive layer on the release liner, and adhering the support to the adhesive layer.

The thickness of the adhesive layer after drying is generally 10 - 250 µm, preferably 40 - 160 µm.

As the support to be used in the present invention, those generally used as a support of an adhesive agent for nail can be used. As the material of the support, for example, polyester such as cellulose acetate, ethylcellulose, polyethylene terephthalate etc., vinyl acetate - vinyl chloride copolymer, plastic polyvinyl chloride, polyurethane, polyolefin, polyvinylidene chloride, aluminum and the like can be mentioned. A sheet made of these, which is in the form of a film, woven fabric, non-woven fabric or paper, is obtained, the sheet as it is or upon lamination can be used as a support of the adhesive agent of the present invention. An aluminum foil may be used as a support.

The thickness of the support is generally 1 - 100 µm, preferably 5 - 50 µm.

As the release liner, synthetic resin film made of polyethylene, polypropylene, polyethylene terephthalate etc., rubber sheet, fabric, non-woven fabric, net, foam sheet, metal foil, laminates thereof and the like can be mentioned. The surface of a release liner may be subjected to a release treatment as necessary, such as silicone treatment, long chain alkyl treatment, fluorine treatment and the like, to improve release property from an adhesive layer. The thickness of the release liner is generally 10 - 200 µm, preferably 25 - 100 µm.

The shape of the adhesive agent for nail of the present invention is not limited and, for example, tape, sheet, artificial nail and the like can be mentioned.

The composition and adhesive agent of the present invention can be preferably used for fixing artificial nail, nail plate protector, ingrown nail corrector etc., therapeutic agents for onychomycosis, hapalonychia, onycholysis, onychophagia and the like, and nailcare products and the like for cosmetic purposes.

### Examples

The present invention is explained in more detail in the following by referring to Examples, which are not to be construed as limitative.

The retention force of the adhesive agent for nail in each Example was measured according to JIS Z 0237. The value is an average value (N=3) of the time (min) before falling off as measured using a bakelite plate as a test plate, wherein the width of test piece 10 mm, adhesion area 200 mm², load 2.94 N and atmospheric temperature 40°C.

The pressure-sensitive adhesive composition with falling off time of less than 3 min showed liner release failure, glue remainder on removal from the nail, cold flow during preservation.

The number of days of adhesion of the adhesive agent to the nail in each Example is an average value of the days before falling off of a pressure-sensitive adhesive composition after adhesion to the nail plate of the toes of six volunteers carefully not to extend from the nail plate. When the adhesive agent did not fall off for 7 days, the test was stopped then.

### Example 1

A solution of a condensation polymer of a silicone resin and a silicone rubber (BIO-PSA 7-4502, manufactured by DOW CORNING) was applied to a PET liner treated with a fluorine release agent, and dried by heating to form an adhesive layer (thickness 60 mm). This was adhered to a 12 mm thick PET film as a support to give an adhesive agent for nail. The results of the measurement of the number of days of adhesion to the nail are shown in Table 1.

### Comparative Example 1

In the same manner as in Example 1 except that a solution of an acrylic adhesive (DURO-TAK 387-2516, manufactured by NATIONAL STARCH&CHEMICAL) was used instead of the solution of the condensation, an adhesive agent for nail was prepared. The results of the measurement of the number of days of adhesion to the nail are shown in Table 1.

### Comparative Example 2

In the same manner as in Example 1 except that a solution of a rubber adhesive was used instead of the solution of a condensation polymer, an adhesive agent for nail was prepared. The solution of the rubber adhesive was obtained by dissolving medium molecular weight polyisobutylene (10 parts, OPPANOL B80, manufactured by BASF), low molecular weight polyisobutylene (15 parts, OPPANOL B12, manufactured by BASF), and polybutene(HV-300, manufactured by Nippon Petrochemicals Co., Ltd.) in 15 parts of toluene. The results of the measurement of the number of days of adhesion to the nail are shown in Table 1.

**Table 1**

| | Adhesive | number of days of adhesion to the nail(DAY) |
|---|---|---|
| Example 1 | silicone adhesive | >7 |
| Comparative Example 1 | acrylic adhesive | 4 |
| Comparative Example 2 | rubber adhesive | 3 |

The adhesive agent for nail of the present invention using a condensation polymer of a silicone resin and a silicone rubber was superior in adhesion to the nail plate.

### Example 2

A solution of a condensation polymer (95 parts, solid content, BIO-PSA 7-4502) of a silicone resin and a silicone rubber was mixed with silicone oil (5 parts, Q7-9120 SILICONE FLUID 100 cSt, manufactured by DOW CORNING) as an additive, and an adhesive agent for nail was prepared in the same manner as in Example 1. The results of the measurement of the number of days of adhesion to the nail and the retention force are shown in Table 2.

### Example 3

In the same manner as in Example 2 except that isopropyl myristate was used instead of silicone oil, an adhesive agent for nail was prepared. The results of the measurement of the number of days of adhesion to the nail and the retention force are shown in Table 2.

### Example 4

In the same manner as in Example 2 except that propyleneglycol dicaprylate was used instead of silicone oil, an adhesive agent for nail was prepared. The results of the measurement of the number of days of adhesion to the nail and the retention force are shown in Table 2.

### Example 5

In the same manner as in Example 2 except that 2-octyldodecanol was used instead of silicone oil, an adhesive agent for nail was prepared. The results of the measurement of the number of days of adhesion to the nail and the retention force are shown in Table 2.

**Table 2**

| | additive | number of days of adhesion to the nail (DAY) | retention force (min) |
|---|---|---|---|
| Example 2 | silicone oil | >7 | 412 |
| Example 3 | isopropyl myristate | >7 | 2 |
| Example 4 | propyleneglycol dicaprylate | >7 | 15 |
| Example 5 | 2-octyldodecanol | >7 | 2 |

The adhesive agent for nail of the present invention was superior in adhesion to the nail plate even when it contained various additives. However, some of them showed glue remainder on removal from the nail plate. The adhesive agent for nail, which showed glue remainder, had a retention force for about 2 min.

### Example 6

A mixed solution (95 parts, solid content, BIO-PSA 7-4602/BIO-PSA 7-4402=50/45 (w/w)) of two kinds of condensation polymers (BIO-PSA 7-4602: resin/rubber ratio=55/45 (w/w), BIO-PSA 7-4402: resin/rubber ratio=65/35 (w/w), both manufactured by DOWCORNING) having different composition ratios of a silicone resin and a silicone rubber was mixed with propyleneglycol dicaprylate (5 parts) and an adhesive agent for nail was prepared in the same manner as in Example 1. The results of the measurement of the number of days of adhesion to the nail and the retention force are shown in Table 3.

### Example 7

In the same manner as in Example 6 except that the mixing ratio of the solution of the condensation polymer was set to BIO-PSA 7-4602/BIO-PSA 7-4402=30/65 (solid content, w/w), an adhesive agent for nail was prepared. The results of the measurement of the number of days of adhesion to the nail and the retention force are shown in Table 3.

### Example 8

In the same manner as in Example 6 except that the mixing ratio of the solution of the condensation polymer was set to BIO-PSA 7-4602/BIO-PSA 7-4402=10/85 (solid content, w/w), an adhesive agent for nail was prepared.

The results of the measurement of the number of days of adhesion to the nail and the retention force are shown in Table 3.

**Table 3**

| | mixing ratio (w/w) 7-4602/7-4402 | number of days of adhesion to the nail (DAY) | retention force (min) |
|---|---|---|---|
| Example 6 | 50/45 | >7 | 5 |
| Example 7 | 30/65 | >7 | 9 |
| Example 8 | 10/85 | >7 | 17 |

The adhesive properties such as retention force etc. could be controlled by the use of a mixture of condensation polymers having different composition ratios of a silicone resin and a silicone rubber. These adhesive agents for nail showed superior adhesion to the nail plate.

### Example 9

A solution of a condensation polymer (90 parts, solid content, BIO-PSA 7-4502) was mixed with propyleneglycol dicaprylate (10 parts) and benzoyl peroxide (1.8 parts) and the mixture was applied to a liner. The solvent was dried at a temperature (100 - 150°C) sufficient to produce free radical of benzoyl peroxide and cause simultaneous crosslinking. Finally, a PET film was adhered to give an adhesive agent for nail. The results of the measurement of the number of days of adhesion to the nail and the retention force are shown in Table 4.

**Table 4**

| | Additive | number of days of adhesion to the nail(DAY) | retention force (min) |
|---|---|---|---|
| Example 9 | propyleneglycol dicaprylate 10 parts benzoyl peroxide 1.8 parts | >7 | >1000 |

The retention force of the adhesive agent for nail was markedly improved by forming a new crosslinking structure by radical condensation using peroxide.

### Example 10

A mixed solution (90 parts, solid content, BIO-PSA 7-4502/DURO-TAK 87-2196=2/1 (w/w)) of a solution of a condensation polymer (BIO-PSA 7-4502) and an acrylic adhesive solution (DURO-TAK 87-2196, manufactured by NATIONAL STARCH & CHEMICAL) was mixed with propyleneglycol dicaprylate (10 parts) and, in the same manner as in Example 1, the mixture was applied to a liner, dried and adhered to a PET film. This was aged at 70°C for 48 hr to give an adhesive agent for nail. The results of the measurement of the number of days of adhesion to the nail and the retention force are shown in Table 5.

### Example 11

In the same manner as in Example 10 except that the mixing ratio of the solution of the condensation polymer and the acrylic adhesive solution was set to BIO-PSA 7-4502/DURO-TAK 87-2196=1/1 (solid content, w/w), an adhesive agent for nail was prepared. The results of the measurement of the number of days of adhesion to the nail and the retention force are shown in Table 5.

### Example 12

In the same manner as in Example 10 except that the mixing ratio of the solution of the condensation polymer and the acrylic adhesive solution was set to BIO-PSA 7-4502/DURO-TAK 87-2196=1/2 (solid content, w/w), an adhesive agent for nail was prepared. The results of the measurement of the number of days of adhesion to the nail and the retention force are shown in Table 5.

**Table 5**

| | mixing ratio (w/w) 7-4502/87-2196 | number of days of adhesion to the nail(DAY) | retention force (min) |
|---|---|---|---|
| Example 10 | 2/1 | >7 | 13 |
| Example 11 | 1/1 | >7 | 44 |
| Example 12 | 1/2 | >7 | 86 |

The adhesive agent for nail prepared from a pressure-sensitive adhesive composition containing a condensation polymer and an acrylic adhesive showed more improved retention force as the ratio of the acrylic adhesive increased.

### Example 13

A solution of a condensation polymer (BIO-PSA 7-4502, 90 parts, solid content) was mixed with propyleneglycol dicaprylate (5 parts), and further with neticonazole (5 parts) as an antifungal agent. The mixture was treated in the same manner as in Example 1 to give an adhesive agent for nail. The following drug permeability test was performed with regard to the adhesive agent for nail.

In a drug permeability test, an adhesive agent for nail having a diameter of 6 mm was adhered to the exuviae of African python and the exuviae was set on a permeation test apparatus (automatic flow thru diffusion cell apparatus, manufactured by Vangard International Inc.). The 24 hr cumulative amount of neticonazole permeated through to PBS on the receptor side was measured by liquid chromatography and the average value was determined (N=3).

In addition, the drug residual ratio of the adhesive agent for nail after preservation at 50°C for 3 months was measured by liquid chromatography, and average value was determined (n=3). The results are shown in Table 6.

**Table 6**

| | 24 hr cumulative permeation amount | drug residual ratio |
|---|---|---|
| Example 13 | 2 µg/cm² | 100% |

The adhesive agent for nail of the present invention showed permeation of the drug. In addition, it showed superior drug stability.

### Example 14

In the same manner as in Example 13 except that the solution of the condensation polymer was changed from one (BIO-PSA 7-4502, manufactured by DOW CORNING) containing residual silanol at about 600 ppm to one (BIO-PSA 7-4202, manufactured by DOW CORNING) containing residual silanol at about 125 ppm, an adhesive agent for nail was prepared.

The drug permeability and drug residual ratio after preservation at 50°C for 3 months of the adhesive agent for nail were measured in the same manner as in Example 13.

The results are shown in Table 7.

**Table 7**

| | 24 hr cumulative permeation amount | drug residual ratio |
|---|---|---|
| Example 14 | 18 µg/cm² | 92% |

The use of a condensation polymer, wherein residual silanol had been decreased, resulted in a markedly improved amount of drug permeation.

### Example 15

A mixed solution (95 parts, solid content, BIO-PSA 7-4202/BIO-PSA 7-4502=2/1 (w/w)) of two kinds of condensation polymers (BIO-PSA 7-4502, BIO-PSA 7-4202) having different residual silanol concentrations was mixed with neticonazole (5 parts), and an adhesive agent for nail was obtained in the same manner as in Example 1.

The drug permeability and drug residual ratio after preservation at 50°C for 3 months of the adhesive agent for nail were measured in the same manner as in Example 13. The results are shown in Table 8.

### Example 16

In the same manner as in Example 15 except that the mixing ratio of the two kinds of condensation polymers having different residual silanol concentrations was changed to BIO-PSA 7-4202/BIO-PSA 7-4502=1/1 (solid content, w/w), an adhesive agent for nail was prepared, and drug permeability and drug residual ratio were measured. The results are shown in Table 8.

### Example 17

In the same manner as in Example 15 except that the mixing ratio of two kinds of solutions having different residual silanol concentrations was set to BIO-PSA 7-4202/BIO-PSA 7-4502=1/2 (solid content, w/w), an adhesive agent for nail was prepared, and drug permeability and drug residual ratio were measured. The results are shown in Table 8.

**Table 8**

| | mixing ratio (w/w) 7-4202/7-4502 | 24 hr cumulative permeation amount | drug residual ratio |
|---|---|---|---|
| Example 15 | 2/1 | 6 µg/cm² | 96% |
| Example 16 | 1/1 | 4 µg/cm² | 98% |
| Example 17 | 1/2 | 3 µg/cm² | 99% |

The adhesive agent for nail (Example 14) using a condensation polymer having decreased residual silanol was somewhat inferior in the drug stability, but, as is clear from Table 8, addition of an adhesive having a different residual silanol concentration could balance the amount of drug permeation and drug stability.

### Example 18

A solution of a condensation polymer (BIO-PSA 7-4202, 90 parts, solid content) was mixed with propyleneglycol dicaprylate (5 parts), neticonazole (5 parts), and L(+)-tartaric acid (0.5 part). This was processed in the same manner as in Example 1 to give an adhesive agent for nail.

The drug permeability and drug residual ratio after preservation at 50°C for 3 months of these adhesive agents for nail were measured in the same manner as in Example 13. The results are shown in Table 9.

### Example 19

In the same manner as in Example 18 except that the amount of L(+)-tartaric acid added was set to 1.0 part, an adhesive agent for nail was obtained. The drug permeability and drug residual ratio after preservation at 50°C for 3 months of the adhesive agent for nail were measured. The results are shown in Table 9.

### Example 20

In the same manner as in Example 18 except that 0.5 part of salicylic acid was added instead of L(+)-tartaric acid, an adhesive agent for nail was obtained. The drug permeability and drug residual ratio after preservation at 50°C for 3 months of the adhesive agent for nail were measured. The results are shown in Table 9.

### Example 21

In the same manner as in Example 20 except that the amount of salicylic acid was set to 1.0 part, an adhesive agent for nail was obtained. The drug permeability and drug residual ratio after preservation at 50°C for 3 months of the adhesive agent for nail were measured. The results are shown in Table 9.

**Table 9**

| | organic acid amount added | 24 hr cumulative permeation amount | drug residual ratio |
|---|---|---|---|
| Example 18 | L(+)-tartaric acid 0.5 part | 12 µg/cm² | 96% |
| Example 19 | L(+)-tartaric acid 1.0 | 8 µg/cm² part | 98% |
| Example 20 | salicylic acid 0.5 part | 12 µg/cm² | 94% |
| Example 21 | salicylic acid 1.0 part | 8 µg/cm² | 96% |

It was found that the drug stability is improved by adding an organic acid.

The composition of the present invention and an adhesive agent using the same have superior adhesive force and cohesion, do not fall off easily during use, do not produce glue remainder easily upon peeling off, and can be preferably used for fixation of artificial nail, nail plate protector, ingrown nail corrector etc., and nailcare products and the like for cosmetic purposes. Furthermore, since the composition of the present invention and adhesive agent also show superior drug releasability and drug stability, they can be preferably applied for treating onychomycosis, hapalonychia, onycholysis, onychophagia and the like. Moreover, since the adhesive agent of the present invention is superior in adhesion to the nail, it shows a superior effect in the administration of drug that causes skin irritation by transdermal application.

The present invention also provides use of the composition of the present invention as an adhesive composition for application to a nail. In one embodiment, the use is non-therapeutic. In another embodiment, the use is cosmetic. In a further embodiment, the use is not a method of treatment.

In another embodiment, the present invention provides the composition of the present invention for use in the treatment of the human or animal body. In a preferred embodiment, the composition comprises a drug. Suitable drugs are those described hereinabove.

In another embodiment, the present invention provides use of the composition of the present invention in the manufacture of a medicament for use as an antifungal agent. In a preferred embodiment, the composition comprises a drug. Suitable drugs are those described hereinabove.

## Claims

1. A pressure-sensitive adhesive composition for use in treatment of the human or animal body by application to a nail, wherein the composition comprises a condensation polymer of a silicone resin and a silicone rubber.

2. A composition for use according to claim 1, which comprises not less than two kinds of condensation polymers having different silicone resin/silicone rubber ratios.

3. A composition for use according to claim 1, wherein the condensation polymer has a silicone resin/silicone rubber ratio of 35/65 - 70/30 (w/w).

4. A composition for use according to any one of the preceding claims, comprising a cross-linked mixture of the condensation polymer or condensation polymers and an acrylic adhesive.

5. A composition for use according to any one of the preceding claims, further comprising at least one additive selected from silicone oil, fatty acid ester of monohydric alcohol, fatty acid ester of polyol and higher alcohol.

6. A composition for use according to any one of the preceding claims further comprising a drug.

7. A composition for use according to claim 6, wherein the condensation polymer has a residual silanol concentration of less than 800 ppm.

8. A composition for use according to claim 6 or claim 7, comprising not less than two kinds of condensation polymers having different residual silanol concentrations.

9. A composition for use according to any one of claims 6 to 8, further comprising an organic acid.

10. A composition for use according to claim 9, wherein the organic acid is tartaric acid or salicylic acid.

11. An adhesive agent for use in treatment of the human or animal body by application to a nail, wherein the agent comprises a support and an adhesive layer comprising a composition as defined in claim 1, which is formed on one surface of the support.

12. A non-therapeutic cosmetic use of a pressure-sensitive adhesive composition comprising a condensation polymer of a silicone resin and a silicone rubber as an adhesive composition for application to a nail.

13. A composition for use according to claim 6, wherein the drug penetrates through the nail plate or is absorbed into the body through the nail plate.

14. A composition for use according to claim 6, for use as an agent for the treatment of onychomycosis.

## Patentansprüche

1. Haftkleberzusammensetzung zur Verwendung bei der Behandlung des menschlichen oder tierischen Körpers durch Auftragung auf einen Nagel, wobei die Zusammensetzung ein Kondensationspolymer aus einem Silikonharz und einem Silikonkautschuk umfasst.

2. Zusammensetzung zur Verwendung gemäß Anspruch 1, die nicht weniger als zwei Arten von Kondensationspolymeren mit unterschiedlichen Verhältnissen von Silikonharz zu Silikonkautschuk umfasst.

3. Zusammensetzung zur Verwendung gemäß Anspruch 1, wobei das Kondensationspolymer ein Verhältnis von Silikonharz zu Silikonkautschuk von 35/65 bis 70/30 (w/w) aufweist.

4. Zusammensetzung zur Verwendung gemäß einem der vorstehenden Ansprüche, umfassend ein vernetztes Gemisch des oder der Kondensationspolymere und eines Acrylklebers.

5. Zusammensetzung zur Verwendung gemäß einem der vorstehenden Ansprüche, weiterhin umfassend wenigstens ein Additiv, das aus Silikonöl, Fettsäureester eines einwertigen Alkohols, Fettsäureester eines Polyols und höherem Alkohol ausgewählt ist.

6. Zusammensetzung zur Verwendung gemäß einem der vorstehenden Ansprüche, weiterhin umfassend einen Wirkstoff.

7. Zusammensetzung zur Verwendung gemäß Anspruch 6, wobei das Kondensationspolymer eine Restsilanolkonzentration von weniger als 800 ppm aufweist.

8. Zusammensetzung zur Verwendung gemäß Anspruch 6 oder 7, die nicht weniger als zwei Arten von Kondensationspolymeren mit unterschiedlichen Restsilanolkonzentrationen umfasst.

9. Zusammensetzung zur Verwendung gemäß einem der Ansprüche 6 bis 8, die weiterhin eine organische Säure umfasst.

10. Zusammensetzung zur Verwendung gemäß Anspruch 9, wobei es sich bei der organischen Säure um Weinsäure oder Salicylsäure handelt.

11. Klebemittel zur Verwendung bei der Behandlung des menschlichen oder tierischen Körpers durch Auftragung auf einen Nagel, wobei das Mittel einen Träger und eine Kleberschicht mit einer Zusammensetzung gemäß Anspruch 1, die auf einer Fläche des Trägers ausgebildet ist, umfasst.

12. Nichttherapeutische kosmetische Verwendung einer Haftkleberzusammensetzung, die ein Kondensationspolymer aus einem Silikonharz und einem Silikonkautschuk umfasst, als Kleberzusammensetzung zur Auftragung auf einen Nagel.

13. Zusammensetzung zur Verwendung gemäß Anspruch 6, wobei der Wirkstoff durch die Nagelplatte dringt oder durch die Nagelplatte hindurch in den Körper resorbiert wird.

14. Zusammensetzung zur Verwendung gemäß Anspruch 6 zur Verwendung als Mittel für die Behandlung von Nagelpilz.

## Revendications

1. Composition adhésive sensible à la pression pour utilisation dans le traitement du corps humain ou animal par application sur un ongle, laquelle composition comprend un polymère de condensation d'une résine siliconée et d'un caoutchouc siliconé.

2. Composition pour utilisation selon la revendication 1, qui comprend au moins deux types de polymères de condensation ayant des rapports résine siliconée/caoutchouc siliconé différents.

3. Composition pour utilisation selon la revendication 1, dans laquelle le polymère de condensation a un rapport résine siliconée/caoutchouc siliconé de 35/65 à 70/30 (p/p).

4. Composition pour utilisation selon l'une quelconque des revendications précédentes, comprenant un mélange réticulé du polymère de condensation ou des polymères de condensation et d'un adhésif acrylique.

5. Composition pour utilisation selon l'une quelconque des revendications précédentes, comprenant en outre au moins un additif choisi parmi une huile siliconée, un ester d'acide gras et d'alcool monovalent, un ester d'acide gras et de polyol et un alcool supérieur.

6. Composition pour utilisation selon l'une quelconque des revendications précédentes, comprenant en outre un médicament.

7. Composition pour utilisation selon la revendication 6, dans laquelle le polymère de condensation a une concentration de silanol résiduel inférieure à 800 ppm.

8. Composition pour utilisation selon la revendication 6 ou la revendication 7, comprenant au moins deux types de polymères de condensation ayant des concentrations de silanol résiduel différentes.

9. Composition pour utilisation selon l'une quelconque des revendications 6 à 8, comprenant en outre un acide organique.

10. Composition pour utilisation selon la revendication 9, dans laquelle l'acide organique est l'acide tartrique ou l'acide salicylique.

11. Agent adhésif pour utilisation dans le traitement du corps humain ou animal par application sur un ongle, lequel agent comprend un support et une couche adhésive comprenant une composition telle que définie dans la revendication 1, qui est formée sur une surface du support.

12. Utilisation cosmétique non thérapeutique d'une composition adhésive sensible à la pression comprenant un polymère de condensation d'une résine siliconée et d'un caoutchouc siliconé en tant que composition adhésive pour application sur un ongle.

13. Composition pour utilisation selon la revendication 6, dans laquelle le médicament pénètre à travers la plaque de l'ongle ou est absorbé dans le corps par l'intermédiaire de la plaque de l'ongle.

14. Composition pour utilisation selon la revendication 6, pour utilisation en tant qu'agent pour le traitement de l'onychomycose.
